# EUROPÄISCHE PATENTANMELDUNG

(11) **EP 2 634 583 A1**
(43) Veröffentlichungstag der Anmeldung: **04.09.2013**
(21) Anmeldenummer: 12157223.4
(22) Anmeldetag: 28.02.2012
(51) Int. Cl.: G01N 33/68

(54) **Screening-Methode zum Auffinden von Proben mit Antiphospholipid-Antikörpern**

(71) Anmelder: Siemens Healthcare Diagnostics Products GmbH, 35041 Marburg (DE)
(72) Erfinder: Patzke, Juergen, 35043 Marburg (DE)

(57) **Zusammenfassung**

Die Erfindung liegt auf dem Gebiet der Gerinnungsdiagnostik und betrifft ein Screening-Verfahren zum Auffinden von Proben, die Antiphospholipid-Antikörper (Lupus Antikoagulanzien) enthalten, wobei die plättchenaggregations-stimulierende Wirkung der Antiphospholipid-Antikörper in vitro bestimmt wird.

## Beschreibung

Die Erfindung liegt auf dem Gebiet der Gerinnungsdiagnostik und betrifft ein Screening-Verfahren zum Auffinden von Proben, die Antiphospholipid-Antikörper enthalten.

Das Antiphospholipid-Syndrom (APS) ist eine der häufigsten Autoimmunerkrankungen und ruft Thrombosen, wiederkehrende Fehlgeburten sowie Schwangerschaftskomplikationen hervor. Ursächlich für ein Antiphospholipid-Syndrom sind die sogenannten Antiphospholipid-Antikörper (APA), die an anionische Phospholipide, an Proteine oder an Protein/ Phospholipid-Komplexe binden. Die Antiphospholipid-Antikörper, die mit bestimmten Proteinen und Phospholipiden Komplexe bilden, stellen eine sehr heterogene Gruppe von Autoantikörpern dar, die gegen eine Vielzahl von Antigenen gerichtet sein können, wie z.B. gegen das Apolipoprotein β2-Glykoprotein I (β2GPI), Cardiolipin, Prothrombin, Protein C, Protein S Annexin V, Thrombomodulin, Faktor XII und andere, sowie gegen Komplexe dieser Proteine mit Phospholipiden.

Die sogenannten Lupus Antikoagulanzien sind Antiphospholipid-Antikörper, die definitionsgemäß die Gerinnungszeiten von bestimmten Gerinnungstesten, z.B. von der aPTT verlängern. Paradox ist, dass die Lupus Antikoagulanzien in vitro eine Hemmung der Gerinnungsreaktion bewirken, während in vivo eine erhöhte Gerinnungsreaktion (Hyperkoagulabilität) mit dem Antiphospholipid-Syndrom (APS) einhergeht. Es gibt allerdings auch Antiphospholipid-Antikörper, die von diesen Testen nicht erfasst werden und die trotzdem prothrombotisch wirksam sind.

Es ist noch nicht abschließend geklärt, wie die Antiphospholipid-Antikörper ihre prothrombotische Wirkung in vivo entfalten. Ein Wirkungsmechanismus scheint über die Aktivierung der Blutplättchen zu verlaufen (Urbanus, R.T. et al., Platelets and the antiphospholipd syndrome. Lupus 2008 Oct; 17 (10) : 888-94).

Die Labordiagnose eines Antiphospholipid-Syndroms (APS) wird durch die Heterogenität der Antiphospholipid-Antikörper erschwert. Zum direkten Nachweis der Antikörper werden immunologische Verfahren angewandt. Hierbei werden allerdings auch viele Antikörper erfasst, die in vivo nicht prothrombotisch wirksam sind. Zum indirekten Nachweis der Antikörper werden Gerinnungsteste angewandt. Besonders Teste, die auf der Bestimmung der DRVVT (Dilute Russell's Viper Venom Time) basieren, zeigen eine relativ gute Korrelation zur prothrombotischen Wirksamkeit der Antiphospholipid-Antikörper. Die Lupus-Diagnostik ist sehr aufwändig, weil jede Patientenprobe mehrere Untersuchungsschritte durchlaufen muss (zur Übersicht: Devreese, K. and Hoylaerts, M.F., Challenges in the diagnosis of the antiphospholipid syndrome. Clin. Chem. 2010, 56(6): 930-940).

Es wäre daher wünschenswert über einen Screening-Test zu verfügen, der es ermöglicht, Proben zu identifizieren, die mit hoher Wahrscheinlichkeit prothrombotisch wirksame Antiphospholipid-Antikörper enthalten. Dies hätte den Vorteil, dass nur solche Proben mit den aufwändigen spezifischen Nachweisverfahren untersucht werden müssten, für die sich mittels Screening-Test ein erster Anhaltspunkt für das Vorliegen von prothrombotischen Antiphospholipid-Antikörpern ergeben hat.

Der vorliegenden Erfindung lag daher die Aufgabe zugrunde ein Verfahren bereit zu stellen, das es ermöglicht, Proben zu identifizieren, die mit hoher Wahrscheinlichkeit prothrombotische Antiphospholipid-Antikörper enthalten.

Die Aufgabe wird durch ein Verfahren mit den folgenden Schritten gelöst:
a) Vermischen einer Probe eines Patienten mit einem plättchenhaltigen Reagenz zu einem Testansatz,
b) Zugabe eines Plättchenaktivators zum Testansatz, und
c) Messen der Plättchenaggregation in dem Testansatz.

Eine gegenüber dem Normal erhöhte Plättchenaggregation im Testansatz zeigt das Vorhandensein von prothrombotischen Antiphospholipid-Antikörpern in der Probe des Patienten an.

Es wurde gefunden, dass in einer Mischung aus Patientenprobe und einem plättchenhaltigen Reagenz prothrombotische Antiphospholipid-Antikörper, die in der Probe des Patienten enthalten sind, die Aggregation der Plättchen verstärken. Zwar ist der Test nicht spezifisch für prothrombotische Antiphospholipid-Antikörper, denn auch andere plättchenaggregations-stimulierende Faktoren, wie z.B. ein erhöhter Thrombinspiegel, können zu einer verstärkten Plättchenaggregation führen. Nichtsdestotrotz können Proben, die in dem Test die Aggregation der Plättchen nicht verstärken, aussortiert werden und brauchen nicht mit Folgetesten zur spezifischen Bestimmung von Antiphospholipid-Antikörpern untersucht zu werden.

Der Begriff "prothrombotische Antiphospholipid-Antikörper" bezeichnet Antiphospholipid-Antikörper, die in vitro die Gerinnungszeit einer Plasmaprobe verlängern. Eine Verlängerung der Gerinnungszeit kann z.B. mit Hilfe eines DRVVT-basierten Gerinnungstests bestimmt werden. Allerdings ist die Sensitivität solcher "Lupus Antikoagulanz"-Teste nicht ausreichend, so dass manche prothrombotische Antiphospholipid-Antikörper nicht erkannt werden. Es ist wahrscheinlich, dass das erfindungsgemässe Verfahren eine bessere Sensitivität aufweist, weil dabei auch in vitro die prothrombotische Wirkung gemessen wird. Anstelle der gerinnungshemmenden Wirkung wird in vitro die tatsächliche Plättchenaggregations-fördernde Wirkung gemessen.

Der Begriff "Probe des Patienten" umfasst in erster Linie Körperflüssigkeiten, insbesondere plättchenreiches Plasma, plättchenarmes Plasma, Serum und Vollblut.

Der Begriff "plättchenhaltiges Reagenz" umfasst Suspensionen, die funktionsfähige, d.h. aggregationsfähige Plättchen (Thrombozyten) humanen Ursprungs enthalten. Es kann sich z.B. um eine Suspension von isolierten Plättchen in einer Pufferlösung oder handeln. Unter dem Begriff "Suspensionen" sind auch Resuspensionen von gefriergetrockneten Plättchen zu verstehen. Alternativ kann es sich um plättchenreiches Plasma oder Vollblut eines Spenders handeln oder um einen Plasmapool mehrerer Spender.

Unter dem Begriff "Plättchenaktivator" ist eine Substanz zu verstehen, die die Aggregation von Thrombozyten induzieren kann. Geeignete Plättchenaktivatoren sind beispielsweise ADP (Adenosin-5'-diphosphat), Kollagen, Epinephrin, Arachidonsäure, Ristocetin und Thrombin. Es können auch Kombinationen von Plättchenaktivatoren zum Testansatz hinzugegeben werden, beispielsweise eine Kombination von Kollagen und ADP (Kol/ADP) oder eine Kombination von Kollagen und Epinephrin (Kol/Epi).

Die bevorzugte Konzentration von Epinephrin liegt zwischen 1 und 20 µmol/L im Testansatz. Die bevorzugte Konzentration von ADP liegt zwischen 1 und 0,2-30 µmol/L im Testansatz, besonders bevorzugt von 0,2 -10 µmol/L im Testansatz.

Die quantitative Bestimmung der Plättchenaggregation im Testansatz, die mit der plättchenaggregations-stimulierenden Wirkung der in der Patientenprobe enthaltenen Antiphospholipid-Antikörper korreliert, kann über die Messung der Streulichtintensität (nephelometrisch) oder über die Messung der Trübung des Testansatzes (turbidimetrisch) erfolgen.

Bevorzugterweise wird die Geschwindigkeit der Plättchenaggregation im Testansatz gemessen. Als besonders aussagekräftig hat sich die Messung der Plättchenaggregationsgeschwindigkeit innerhalb des Zeitraums von 12 bis 50 Sekunden nach Zugabe eines Plättchenaktivators zum Testansatz erwiesen. Alternativ kann die maximale Plättchenaggregationsgeschwindigkeit bestimmt werden.

In einer anderen Ausführungsform des erfindungsgemäßen Verfahrens findet die Messung der Plättchenaggregation unter Flussbedingungen und damit in Gegenwart von hohen Scherkräften statt. Ein solches Testprinzip ist beispielsweise im Platelet Function Analyzer System (PFA-100^{®}, PFA-200, Siemens Healthcare Diagnostics GmbH, Marburg, Deutschland) verwirklicht. Dieses Messverfahren eignet sich insbesondere dann, wenn die Patientenprobe und/oder das plättchenhaltige Reagenz aus Vollblut bestehen.

Zur Simulation der Flussbedingungen und der Scherkräfte, wie sie in kleineren arteriellen Blutgefäßen herrschen, wird in einer speziellen Messzelle ein Unterdruck von etwa -40mbar erzeugt und das Citratvollblut, dass sich in einem Probenreservoir befindet, strömt durch eine Kapillare, die einen Durchmesser von etwa 100-200µm hat. Die Kapillare mündet in eine Messkammer, die mit einem Trennelement, z.B. mit einer Membran, abgeschlossen ist, welche eine kapillare zentrale Öffnung (Apertur) enthält, durch die das Blut aufgrund des Unterdrucks durchtritt. In den meisten Fällen ist die Membran zumindest in dem Bereich um die Apertur mit einem oder mehreren Aktivatoren, die die Plättchenaggregation induzieren, versetzt, so dass das vorbeiströmende Blut mit den aggregationsinduzierenden Substanzen im Bereich der Apertur in Kontakt kommt. Infolge der induzierten Adhäsion und Aggregation der Thrombozyten bildet sich im Bereich der Apertur ein Thrombozytenpfropf (Thrombus), der die Membranöffnung verschließt und den Blutfluss stoppt. In diesem System wird üblicherweise die Zeit gemessen, die bis zum Verschluss der Membranöffnung benötigt wird. Diese sogenannte Verschlusszeit (CT) korreliert mit der Funktionsfähigkeit der Thrombozyten. Eine Messzelle zur Verwendung in einem Verfahren zu Bestimmung der Thrombozytenfunktion anhand der Verschlusszeit ist z.B. in dem Patentdokument WO 97/34698 beschrieben. Bevorzugte Messzellen sind mit einer Membran ausgestattet sind, die mit Kollagen (Kol) und zusätzlich entweder mit ADP oder mit Epinephrin (Epi) beschichtet ist oder die mit ADP und Prostaglandin E1 (PGE1) beschichtet ist. Verschiedene Trennelemente sowie deren Herstellung und Verwendung sind z.B. in dem Patentdokument EP-B1-716744 oder in EP-A1-1850134 beschrieben.

In einer bevorzugten Ausführungsform des erfindungsgemäßen Verfahrens umfasst die Messung der Thrombozytenaktivität also das Durchleiten der Probe, die mit einem plättchenhaltigen Reagenz vermischt wurde, durch eine Kapillare und anschließend durch eine Öffnung eines Trennelements und Messung der Zeit, die für die Bildung eines Thrombozytenpfropfs an der Öffnung des Trennelements bis zum Verschluss der Öffnung benötigt wird.

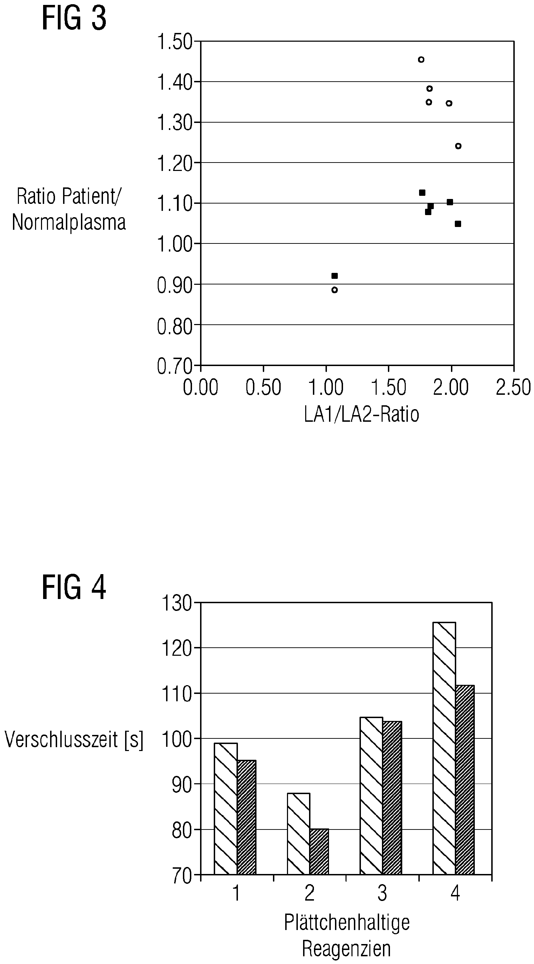

Wenn prothrombotische Antiphospholipid-Antikörper in der Probe enthalten sind, bewirkt die Zugabe der Probe zum plättchenhaltigen Reagenz, welches bevorzugt aus Vollblut besteht, eine Verkürzung der Verschlusszeit im Vergleich zu der Zugabe einer normalen Probe, die keine prothrombotischen Antiphospholipid-Antikörper enthält(Figur 4).

Weiterhin bevorzugt ist die Bestimmung eines Ratios (Quotienten) des Testergebnisses der Patientenprobe zu dem Testergebnis einer Normalprobe (Norm). Eine Normalprobe kann eine Probe eines gesunden Spenders oder ein Pool von Proben mehrerer gesunder Spender sein. Dazu wird in einem zweiten Testansatz, bei dem eine Normalprobe, die keine Antiphospholipid-Antikörper enthält, mit dem plättchenhaltigen Reagenz zu einem Testansatz vermischt , die normale Plättchenaggregation wird gemessen, und die Testergebnisse werden ins Verhältnis gesetzt. Ein Verhältnis größer 1.0 von der im Testansatz der Patientenprobe gemessenen Plättchenaggregation zu der normalen Plättchenaggregation zeigt das Vorhandensein von prothrombotischen Antiphospholipid-Antikörpern in der Patientenprobe an.

### Figurenbeschreibung

### Figur 1

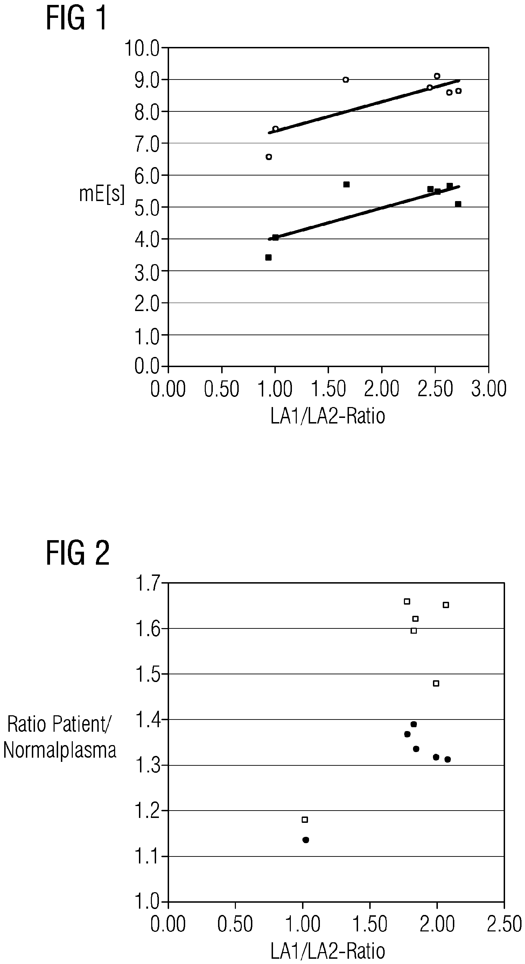

Figur 1 zeigt die Geschwindigkeit der Epinephrin-induzierten Plättchenaggregation (mE/s) in einer Probe eines offensichtlich gesunden Spenders in einer Normalplasma-Pool-Probe und in Proben von 5 Patienten mit Antiphospholipid-Syndrom in Abhängigkeit von dem LA1/LA2-Ratio. Es wurden zwei verschiedene plättchenhaltige Reagenzien eingesetzt: PRP-Reagenz A (Kreise) und PRP-Reagenz B (Quadrate). Proben von Patienten mit Antiphospholipid-Syndrom (LA1/LA2 größer 1) zeigen gegenüber den Normalproben (LA1/LA2 = 1) eine verstärkte Plättchenaggregation.

### Figur 2

Figur 2 zeigt den Quotienten aus der Geschwindigkeit der Epinephrin-induzierten Plättchenaggregation in einer Probe eines offensichtlich gesunden Spenders und in Proben von 5 Patienten mit Antiphospholipid-Syndrom und der Geschwindigkeit der Epinephrin-induzierten Plättchenaggregation in einer Normalplasma-Pool-Probe (Ratio Patient/Normalplasma) in Abhängigkeit von dem LA1/LA2-Ratio. Es wurden zwei verschiedene plättchenhaltige Reagenzien eingesetzt: PRP-Reagenz A (Kreise) und PRP-Reagenz B (Quadrate). Proben von Patienten mit Antiphospholipid-Syndrom (LA1/LA2 größer 1) zeigen gegenüber den Normalproben (LA1/LA2 = 1) einen erhöhten Ratio Patient/Normalplasma.

### Figur 3

Figur 3 zeigt den Quotienten aus der Geschwindigkeit der ADP-induzierten Plättchenaggregation in einer Probe eines offensichtlich gesunden Spenders und in Proben von 5 Patienten mit Antiphospholipid-Syndrom und der Geschwindigkeit der ADP-induzierten Plättchenaggregation in einer Normalplasma-Pool-Probe (Ratio Patient/Normalplasma) in Abhängigkeit von dem LA1/LA2-Ratio. Es wurden zwei verschiedene plättchenhaltige Reagenzien eingesetzt: PRP-Reagenz C (Kreise) und PRP-Reagenz D (Quadrate). Proben von Patienten mit Antiphospholipid-Syndrom (LA1/LA2 größer 1) zeigen gegenüber den Normalproben (LA1/LA2 = 1) einen erhöhten Ratio Patient/Normalplasma.

### Figur 4

Figur 4 zeigt, dass Antiphospholipid-Antikörper in einer Probe (Lupus-Plasma-Pool, fein schraffierte Balken) verglichen mit einer Probe (Normalplasma-Pool, grob schraffierte Balken), die keine Antiphospholipid-Antikörper enthält, eine Verkürzung der Verschlusszeit im Platelet Function Analyzer System bewirken. Es wurden vier verschiedene plättchenhaltige Reagenzien (1-4) aus frischem Vollblut verschiedener gesunder Spender eingesetzt.

### Beispiele

### Beispiel 1: Messung der plättchenaggregations-stimulierenden Wirkung von Antiphospholipid-Antikörpern in Patientenplasma

Als Patienten-Plasma wurden Proben von 5 Patienten mit Antiphospholipid-Syndrom, eine Probe eines offensichtlich gesunden Spenders und ein Normalplasma-Pool (Kontrollplasma N, Siemens Healthcare Diagnostics Products GmbH, Marburg, Deutschland) eingesetzt.

Als plättchenhaltiges Reagenz wurde plättchenreiches Humanplasma (PRP-Reagenz) verwendet, das aus frischem Blut von offensichtlich gesunden Blutspendern durch Zentrifugation (180 g, 10 min) hergestellt worden war. Es wurden 4 plättchenhaltige Reagenzien (A, B, C, D) aus Blut von 4 unterschiedlichen Spendern hergestellt.

135 µL des PRP-Reagenzes wurden mit 40 µL Patienten-Plasma vermischt und bei 37 °C inkubiert. Nach 5 Minuten wurden 15 µL Epinephrin-Lösung (100 µmol/L Epinephrin) oder 15 µL ADP-Lösung (12 µmol/L ADP) zur Aktivierung der Plättchen zugegeben, und die Extinktion des Testansatzes wurde bei 620 nm turbidimetrisch gemessen. Als Maß für die Plättchenaggregation wurde die Extinktionsänderung in dem Zeitraum von 12-50 Sekunden (mE/Sekunde) nach Zugabe des Plättchenaktivators bestimmt.

Parallel wurden alle Proben mit Hilfe eines kommerziell erhältlichen Tests auf Basis eines DRVVT-Gerinnungstestes zum Nachweis von Lupus Antikoagulanzien untersucht (Screening Reagenz LA 1 / Bestätigungsreagenz LA 2, Siemens Healthcare Diagnostics Products GmbH). Das sogenannte LA1/LA2-Ratio gesunder Spender ist nahe 1. Ist das Ratio LA1/LA2 deutlich höher als 1, spricht dies sehr stark für eine Erkrankung am Antiphospholipid-Syndrom. Die Stärke der Erkrankung korreliert mit der Höhe des LA1/LA2-Ratios.

Da plättchenreiches Plasma von verschiedenen Spendern, das hier als plättchenhaltiges Reagenz verwendet wurde, in seiner Aggregationsgeschwindigkeit relativ stark variieren kann, wurden die Ergebnisse der Patienten-Plasmen durch Bildung des Quotienten ("Ratio") aus Patienten-Plasma/Normalplasma-Pool normalisiert (Figuren 2 und 3). Der normalisierte Quotient (Ratio) von Proben von Spendern mit Antiphospholipid-Syndrom ist deutlich höher als der von normalen Spendern, welcher nahe 1 liegt (Tabelle 1).

Die Ergebnisse sind in den Figuren 1 bis 3 dargestellt.

**Tabelle 1**

| **PRP- Reagenz** | **Aktivator** | **Normale Probe** | **Proben von Spendern mit Antiphospholipid-Syndrom** | | | |
|---|---|---|---|---|---|---|
| B | Epinephrin | 1.18 | 1.48 | 1.66 | 1.60 | 1.65 |
| C | ADP | 0.88 | 1.34 | 1.45 | 1.35 | 1.24 |

### Beispiel 2: Messung der Verschlusszeit-verkürzenden Wirkung im Platelet Function Analyzer von Antiphospholipid-Antikörpern in Patientenplasma

Mit Hilfe des Platelet Function Analyzer Systems (PFA-100^{®}, PFA-200, Siemens Healthcare Diagnostics Products GmbH) wird üblicherweise in Vollblutproben die primäre Hämostase unter Flussbedingungen und damit in Gegenwart von hohen Scherkräften gemessen. Als Maß für die Thrombozytenfunktion wird in diesem System die Zeit gemessen, die bis zum Verschluss einer Membranöffnung in einer speziellen Messzelle benötigt wird (Verschlusszeit). Verlängerte Verschlusszeiten weisen auf das Vorliegen einer Thrombozytendysfunktion im Sinne einer verminderten Aggregationsfähigkeit hin. Verkürzte Verschlusszeiten weisen auf das Vorliegen einer Thrombozytendysfunktion im Sinne einer erhöhten Aggregationsfähigkeit hin.

Als Patienten-Plasma wurden ein Plasma-Pool aus mehreren Plasmen von Patienten mit Antiphospholipid-Syndrom (Lupus-Plasma-Pool mit einem LA1/LA2-Ratiowert von 2,53) und ein Normalplasma-Pool (Kontrollplasma N, Siemens Healthcare Diagnostics Products GmbH) eingesetzt.

Als plättchenhaltiges Reagenz wurde frisches Vollblut von offensichtlich gesunden Blutspendern verwendet. Es wurden 4 plättchenhaltige Reagenzien (1, 2, 3, 4) aus Vollblut von 4 unterschiedlichen Spendern hergestellt.

Das plättchenhaltige Reagenz aus frischem Vollblut wurde mit 10 % (v/v) des Patienten-Plasmas gemischt und 10 Minuten inkubiert. Danach wurde der Testansatz in eine PFA-Messzelle pipettiert, die mit einer Membran ausgestattet war, die mit Kollagen (Kol) und mit ADP beschichtet war (Kol/ADP), und es wurde die Verschlusszeit (s) in einem Platelet Function Analyzer gemessen.

Der Plasma-Pool aus mehreren Plasmen von Patienten mit Antiphospholipid-Syndrom (Lupus-Plasma-Pool) zeigte gegenüber dem Normalplasma-Pool verkürzte Verschlusszeiten (siehe Figur 4) .

## Patentansprüche

1. Screening-Verfahren zur Identifizierung einer Probe, die mit hoher Wahrscheinlichkeit prothrombotische Antiphospholipid-Antikörper enthält, wobei das Verfahren folgende Schritte aufweist:
a) Vermischen einer Probe eines Patienten mit einem plättchenhaltigen Reagenz zu einem Testansatz,
b) Zugabe eines Plättchenaktivators zum Testansatz, und
c) Messen der Plättchenaggregation in dem Testansatz,
wobei eine gegenüber dem Normal erhöhte Plättchenaggregation im Testansatz das Vorhandensein von prothrombotischen Antiphospholipid-Antikörpern in der Probe des Patienten anzeigt.

2. Verfahren gemäß Anspruch 1, wobei die Probe des Patienten aus plättchenreichem Plasma, plättchenarmem Plasma, Serum oder Vollblut besteht.

3. Verfahren gemäß einem der vorhergehenden Ansprüche, wobei das plättchenhaltige Reagenz aus einer Suspension von isolierten Plättchen in einer Pufferlösung, aus plättchenreichem Plasma oder Vollblut besteht.

4. Verfahren gemäß einem der vorhergehenden Ansprüche, wobei ein Plättchenaktivator aus der Gruppe ADP, Kollagen, Epinephrin, Arachidonsäure und Thrombin zum Testansatz gegeben wird.

5. Verfahren gemäß einem der Ansprüche 1 bis 4, wobei die Plättchenaggregation im Testansatz turbidimetrisch gemessen wird.

6. Verfahren gemäß einem der Ansprüche 1 bis 4, wobei die Plättchenaggregation im Testansatz nephelometrisch gemessen wird.

7. Verfahren gemäß einem der Ansprüche 1 bis 4, wobei die Plättchenaggregation im Testansatz dadurch gemessen wird, dass
i) der Testansatz durch eine Kapillare und anschließend durch eine Öffnung eines Trennelements geleitet wird, und
ii) die Zeit, die für die Bildung eines Thrombozytenpfropfs an der Öffnung des Trennelements bis zum Verschluss der Öffnung benötigt wird, gemessen wird.

8. Verfahren gemäß einem der Ansprüche 1 bis 4, wobei die Geschwindigkeit der Plättchenaggregation im Testansatz gemessen wird, bevorzugt innerhalb des Zeitraums von 12 bis 50 Sekunden nach Zugabe eines Plättchenaktivators zum Testansatz.

9. Verfahren gemäß einem der Ansprüche 1 bis 4, wobei die maximale Plättchenaggregationsgeschwindigkeit gemessen wird.

10. Verfahren gemäß einem der vorhergehenden Ansprüche, wobei in einem zweiten Testansatz, bei dem eine Normalprobe mit dem plättchenhaltigen Reagenz zu einem Testansatz vermischt wird und die normale Plättchenaggregation gemessen wird und wobei dann die in Schritt c) im Testansatz gemessene Plättchenaggregation dazu ins Verhältnis gesetzt wird und wobei ein Verhältnis größer 1.0 von der in Schritt c) im Testansatz gemessene Plättchenaggregation zu der normalen Plättchenaggregation das Vorhandensein von Antiphospholipid-Antikörpern in der Patientenprobe anzeigt.
